(19) Europäisches Patentamt
European Patent Office
Office européen des brevets

(11) EP 4 388 998 A1

(12) **EUROPEAN PATENT APPLICATION**

(43) Date of publication:
26.06.2024 Bulletin 2024/26

(51) International Patent Classification (IPC):
*A61B 5/145* (2006.01)  *A61B 5/20* (2006.01)
*A61B 5/00* (2006.01)  *A61B 10/00* (2006.01)
*G01N 33/493* (2006.01)

(21) Application number: 22315335.4

(22) Date of filing: 19.12.2022

(52) Cooperative Patent Classification (CPC):
**G01N 33/493; A61B 5/14507; A61B 5/207;
A61B 10/007; G01N 21/8483; G01N 33/54388;**
A61B 5/6891; A61B 2562/0295

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Withings**
**92130 Issy-les-Moulineaux (FR)**

(72) Inventor: **Sounderya, Nagarajan**
**92130 Issy-les-Moulineaux (FR)**

(74) Representative: **Withings IP**
**2, rue Maurice Hartmann**
**92130 Issy-les-Moulineaux (FR)**

(54) **TEST STRIP FOR A URINE OPTICAL ANALYSIS DEVICE CARTRIDGE**

(57)    The invention concerns a test strip (500) for detecting an ion of interest present in urine for a urine optical analysis device cartridge. The test strip comprises:
- a sample pad (510) configured to receive a urine sample,
- a test pad (520) comprising a probe made of a fluorophore conjugated with an ionophore,
- a backing pad (530) on which the sample pad (510) and the test pad (520) are arranged.
    The backing pad (530) is configured to transport the ions of interest across to the test strip from the sample pad (510) towards the test pad (520). The backing pad is made of a porous plastic membrane.

**FIG. 5**

## Description

## Technical Field

The present invention relates to a test strip to detect an ion of interest present in urine. The ion of interest is notably sodium. In a variant, the ion of interest may be potassium or magnesium. The test strip is designed to be arranged in a cartridge designed to be inserted in a station for urine analysis. The station may be installed on the surface of a toilet bowl. The cartridge mounted on the station will be referred to as an analysis device. The station comprises a light source and an optical sensor to carry out an optical analysis on the test strip.

## Background art

The urine of a user may be a source of useful information about the health of the user. Monitoring the ions in the urine of a user may provide information about what the user ingests, of body's waste and excess mineral salts. By regularly monitoring these ions in urine it is possible to control the consumption of these ions in commonly accepted healthy ranges. A sodium concentration in the urine for an individual differing from the commonly accepted healthy ranges may be a symptom of a disorder or a dysfunction of an organ. If such a dysfunction goes undiagnosed and is left untreated, serious health issues may arise. This monitoring may help to diagnose various disorders such as hypertension and cardiovascular diseases for example. A projected one in ten deaths from cardiovascular causes (1.65 million in 2010 according to article "Global sodium consumption and death from cardiovascular causes", https://pubmed.ncbi.nlm.nih.gov/25119608/) are attributed to excess sodium intake.

Moreover, by analyzing the trend of these ions' concentration, it is also possible to detect trend modifications representative of diseases. For example, long-term sodium monitoring may help to detect heart failure.

Twenty-four-hour urine collections are the recommended method of monitoring a person's sodium intake. About 90% of the sodium consumed is excreted in urine and estimated intake from 24h urine collection is not subject to recall bias (se article "Use of Urine Biomarkers to Assess Sodium Intake: Challenges and Opportunities", https://www.ncbi.nlm.nih.gov/pmc/articles/PMC5497310/). However, such a 24h protocol presents a high burden for the individual as he or she has to collect his or her urine during 24h as well as have to deliver it to the hospital in the adapted containers. Therefore, such a protocol seems not to be suitable for a regular monitoring of the sodium concentration of an individual.

In this case, the gold reference method for quantitative sodium and potassium measurements is usually classical electrodes with a potassium membrane or with doped glass for sodium.

Other sodium measurements rely on optical colorimetric detection of the presence of the ion in a biofluid using an ionophore and an indirect pH marker. The ionophore captures the ion and the change in the electronic state liberates a hydrogen ion which triggers a color change in the pH indirect marker. Since the two are not bound to each other the detection suffers from non-specific interactions.

Ghaderinezhad (2020) (Ghaderinezhad, F., Ceylan Koydemir, H., Tseng, D. et al. Sensing of electrolytes in urine using a miniaturized paper-based device. Sci Rep 10, 13620 (2020). https://doi.org/10.1038/s41598-020-70456-6) proposed a smartphone-enabled device for quantifying the concentration of sodium in urine. This device enables the quantification of sodium in artificial urine with fluorescent and colorimetric detection methods. However, the smartphone method is too tedious for an individual to regularly monitor his or her urine sodium concentration.

There is therefore a need for a paper-based fluorescent or colorimetric ion detection method to be carried out with an analysis device as disclosed for example in document WO2021/175909. This document discloses a point-of-care device for urine analysis. The device is to be lodged in a toilet (more precisely on a surface of the toilet bowl) and collects samples of a urine stream before performing an optical analysis. The device comprises a station and a cartridge, which is also called a rotatable support, and which may be removed and replaced from the station. The cartridge contains urinary test strips, that is to say strips coated or impregnated with a reagent that reacts with urine.

The method suggested in Ghaderinezhad is not applicable in such a device. In particular, the paper receiving the probe and the artificial urine sample is too large to be arranged in the cartridge and does not enable a lateral flow analysis. Notably, the paper used is made of cellulose which comprises -OH which may capture the positive ions and thus prevent the migration of ions in the paper. Moreover, the test pads in the paper are prepared by manual dispensing which may not be translated easily to commercial scale preparation of test pads.

## Summary of the disclosure

An aim of the disclosure is therefore to provide a test strip for detecting an ion of interest which may be arranged in a cartridge to carry out urine optical analysis. The test strip aims to provide accurate measurements to provide to an individual a regular, easy, and cost-effective concentration monitoring in urine.

To solve at least one of these issues, the disclosure relates to a test strip for detecting an ion of interest present in urine for a urine optical analysis device cartridge, the test strip comprising: a sample pad configured to receive a urine sample, a test pad comprising a probe made of a fluorophore conjugated with a ionophore, the fluorescence emission intensity of the fluorophore increasing when a ion of interest is binding with the ionophore, and a backing pad on which the sample pad and the test pad are arranged, the backing pad being configured to transport the ions of interest across to the test strip from the sample pad towards the test pad, the backing pad being made of a porous plastic membrane.

In one implementation, the backing pad is made of a porous plastic membrane. It is possible to carry out a concentration measurement of the ion of interest in a urine sample. The sample is received on the sample pad and the urine migrates towards the test pad. The ion of interest present in the urine is captured by the ionophore of the probe, which is specific to the ion of interest, resulting in an energy transfer to the fluorophore of the probe resulting in an increase in the fluorescence emission intensity. This fluorescence is detected and analyzed by an analysis device to determine the spot urine concentration of sodium in the urine sample. The backing pad supports the sample pad and the test pad. The porous plastic membrane enables an efficient transport of the sodium ions contrary to conventional cellulosic materials which cause the ions to bind to the membrane.

In one embodiment, the ion of interest is chosen between: sodium, potassium, and magnesium.

In one embodiment, the test strip extends along a longitudinal direction, the test strip presenting a length inferior to 20 mm, and/or a width inferior to 5 mm.

In one embodiment, the test strip further comprises an excitation optical filter, arranged preferably at the bottom of the test strip, the excitation optical filter being configured to selectively transmit the main excitation wavelength of the fluorophore.

In one embodiment, the test strip further comprises an emission optical filter, arranged preferably on top of the test and reference pad, the emission optical filter being configured to selectively transmit the main emission wavelength of the fluorophore.

In one embodiment, the test pad and the sample pad are fixed to the backing pad with at least an adhesive tape.

In one embodiment, the sample pad is made of a porous plastic membrane.

In one embodiment, the sample pad comprises a buffer able to keep the pH close to 7, in particular between pH 6.5 and 7.5 for a urine sample of pH 5 to 8.

In one embodiment, the test pad is made of cellulose.

In one embodiment, the backing pad is made of a PE and PP hydrophilic membrane.

In one embodiment, the ion of interest is sodium, and the probe is made of a crown ether linked to a fluorophore.

In one embodiment, the ion of interest is potassium.

In one embodiment, the ion of interest is magnesium.

In one embodiment, the test strip further comprises a reference pad arranged on the backing pad, the reference pad comprising the fluorophore without the ionophore.

In one embodiment, the fluorophore is fluorescein.

In one embodiment, the reference pad is made of cellulose.

The invention also concerns a cartridge for a urine optical analysis device, the cartridge comprising a plurality of chambers arranged next to one another in a right circular cylinder shape of at least 80% of a circle with a diameter comprised between 3cm and 10cm, wherein at least a chamber comprises a test strip as described above.

In one embodiment, the cartridge further comprises a pH measuring strip arranged in at least a chamber.

The invention also concerns a urine optical analysis device comprising: the cartridge as described above, and a station, configured to be positioned on a wall of a toilet bowl, the station comprising: a case comprising an annular housing in which the cartridge is at least partially received and is rotatably mounted therein around a rotation axis, an injector, configured to inject urine on the test strip, and an analyzer with a light source and a light sensor, configured to emit and receive light, and to detect a change of fluorescence of the test strip.

The invention also concerns a method to manufacture a test strip as described above, comprising:

- providing a probe and a paper substrate,
- providing a sample pad, and a backing pad made of a porous plastic membrane,
- diluting the probe in a solvent to form a stock solution,
- soaking the paper substrate into the stock solution with shaking,
- drying the probe on the paper substrate by heating it to form a test pad,
- fix the sample pad and the test pad on the backing pad with an adhesive layer.

In one embodiment, the solvent is methanol.

In one embodiment, before the step of fixing the sample pad on the backing pad, the method further comprises the steps of:

- soaking the sample pad in a buffer solution,
- heating the sample pad.

In one embodiment, during the step of heating, the sample pad is heated at a temperature between 35°C and 60 °C.

## Brief Description of Drawings

These features and advantages of the invention will appear more clearly upon reading the following description, provided solely as a non-limiting example, and done in reference to the appended drawings, in which:

[Fig. 1]: Figure 1 shows the overall setup of an analysis device for urine analysis according to an embodiment, as installed on a surface of a toilet bowl,

[FIG. 2]: Figure 2 shows an exploded view of an embodiment of an analysis device, in which the station and the cartridge are visible,

[FIG. 3]: Figure 3 shows a detailed view of a cartridge according to an embodiment,

[FIG. 4]: Figure 4 shows a sectional view of an embodiment of a cartridge and a station, at the location of an optical analyzer of the station,

[FIG. 5]: Figure 5 shows a side view of a test strip according to the invention,

[FIG. 6]: Figure 6 shows a top view of a test strip according to the invention,

[FIG. 7]: Figure 7 is a chemical representation of a probe in the test pad of the test strip,

[FIG. 8]: Figure 8 is a chemical representation of another probe in the test pad of the test strip,

[FIG. 9]: Figure 9 shows two graphs representing the fluorescence as a function of respectively the sodium and the potassium concentration,

[FIG. 10]: Figure 10 shows a graph representing a wet to dry fluorescence ratio as a function of respectively the sodium and the potassium concentration,

[FIG. 11]: Figure 11 is a graph representing the intensity respectively regarding excitation and excitation as a function of the wavelength of the probe,

[FIG. 12]: Figure 12 show a comparison between different materials used as a paper substrate to obtain the test pad of the test strip,

[FIG. 13]: Figure 13 shows a comparison between different materials used as a backing pad of the test strip,

[FIG. 14]: Figure 14 shows a method to manufacture a test strip according to the invention, and

[FIG. 15]: Figure 15 shows a comparison between probe concentration on a paper substrate to obtain the test pad of the test strip.

## Detailed description

The present description introduces different examples of a cartridge usable with a station as disclosed in document WO2021/175909 and WO2021/175944 (publication numbers), hereafter referred to as WO'909 and WO'944. Variations of the stations are presented in any of FR2109383, FR2109384, FR2109391, FR2109392 (filing numbers).

The next paragraphs explain the overall principle of a device for urine analysis, but all the details of WO'909 and WO'933 (and also any of the above-mentioned French filings) are applicable.

## OVERALL DESCRIPTION OF THE STATION AND THE CARTRIDGE

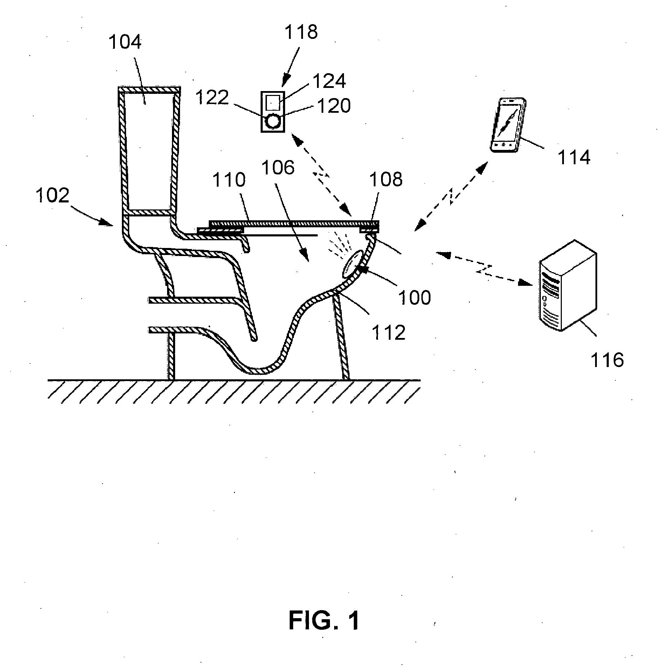

Figure 1 schematically illustrates an analysis device 100 (referred to as the "device 100") for urine analysis as set up in toilets 102. Toilets 102 usually comprise a water tank 104, a bowl 106, a seat 108 and a seat cover 110. The analysis device 100 is arranged in a removable manner in the toilets 102. For example, the analysis device 100 may be easily removed from the toilets to replace a cartridge and then arranged again in the toilets 102. The analysis device 100 is arranged on an internal wall 112 of the bowl 106 of the toilets. The analysis device 100 is placed so that it is usually under a urine stream from a user, such that when a user urinates (typically in a seated position), urine contacts with the analysis device 100. The analysis device 100 may communicate remotely with a remote entity, such as smartphone 114 or a server 116.

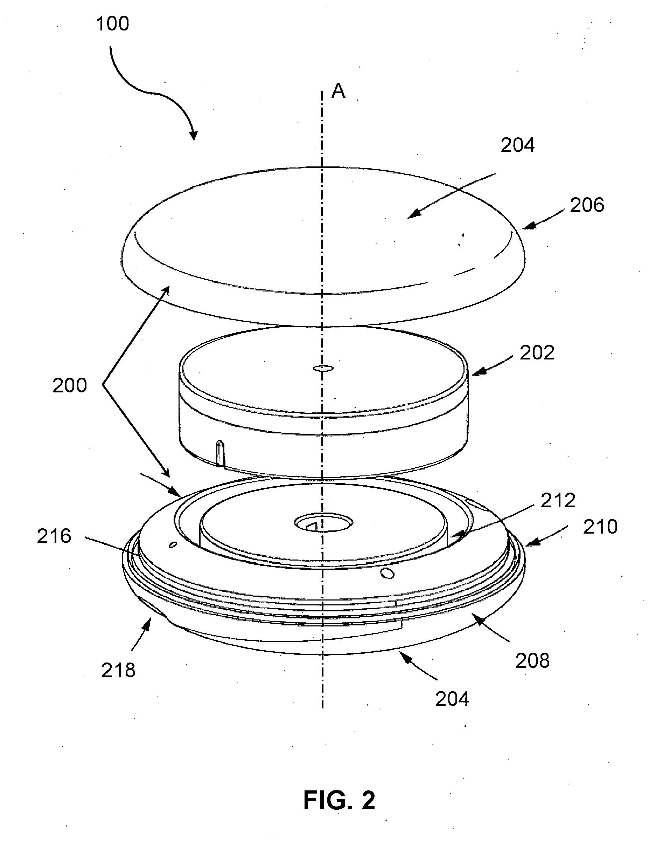

As illustrated in more detail on Figure 2, the analysis device 100 comprises a station 200 and a cartridge 202, mounted in a removable manner from the station 200. Station 200 may comprise a case 204 which may include two shells 206, 208. Case 204 lodges therein a urine testing assembly. Station 200 comprises an annular or ring-shaped housing 212, located inside the case 204, arranged around a rotation axis A. The annular housing 212 is configured to receive at least partially the cartridge 202 mounted in a rotatable manner around the rotation axis A (once in position in the annular housing 212). The cartridge 202 comprises a plurality of test supports which each comprise at least one urine reagent, for instance a dry reagent, the plurality of test supports being arranged along a circle or a circular arc around the rotation axis A. In an embodiment, the test supports are test strips. The test supports may be enclosed, for example individually enclosed, in a chamber.

The annular housing 212 typically extends around 360° and forms a groove configured to receive at least partially the cartridge 202.

Station 200 comprises a collection opening 218, located for example on shell 208. The collection opening 218 collects urine flowing on the surface of the housing 204. A drain opening (not illustrated) is also included to drain the liquid out of the device 100.

The housing 204 may have a diameter, in a direction perpendicular to the rotation axis A, comprising between 50 mm and 150 mm.

The test assembly comprises a pump, an injector and an analyzer. The pump sucks urine from the collection opening 218, then the injector injects the urine on one or more test supports of the cartridge and the analyzer obtains some values of properties (e.g., physical/chemical properties, such as the color) of the test supports after it has contacted the urine. In one embodiment, the analyzer is an optical analyzer configured to analyze optical properties of the test support. The injector and the cartridge may move relatively to each other so that the injector can open (e.g., pierce) the chamber, for example with a needle or needle-like device.

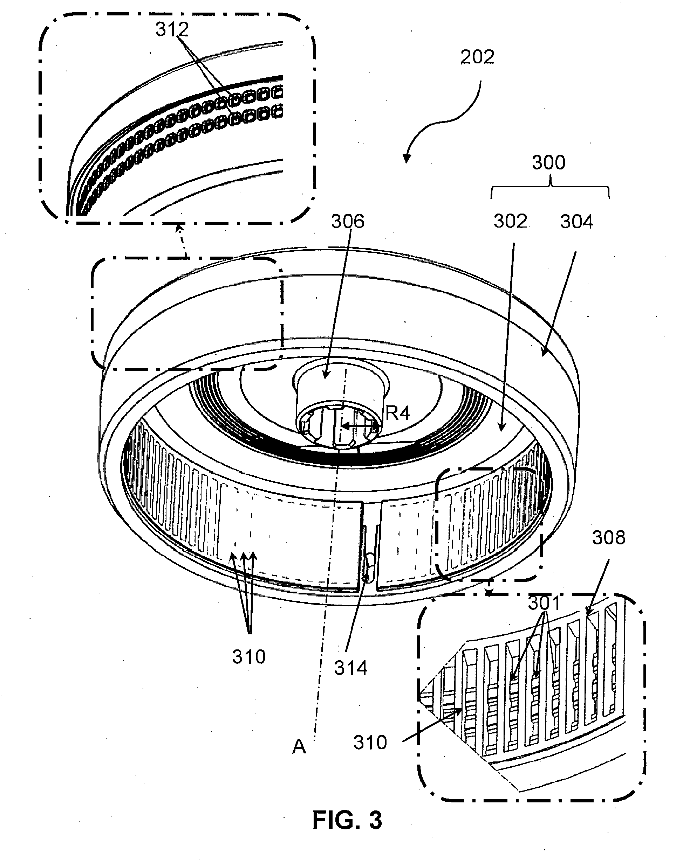

Figure 3 shows an exploded view of the cartridge 202. The cartridge 202 comprises test supports 301 configured to receive urine from the injector. The test support contains a urine reagent that reacts in a specific way when in contact with urine. The cartridge 202 comprises a rotatable support 300, configured to be driven in rotation by the station 200. In normal use of the cartridge 202 and the device 100, the test supports 301 remain attached to the rotatable support and do not move with respect to the latter.

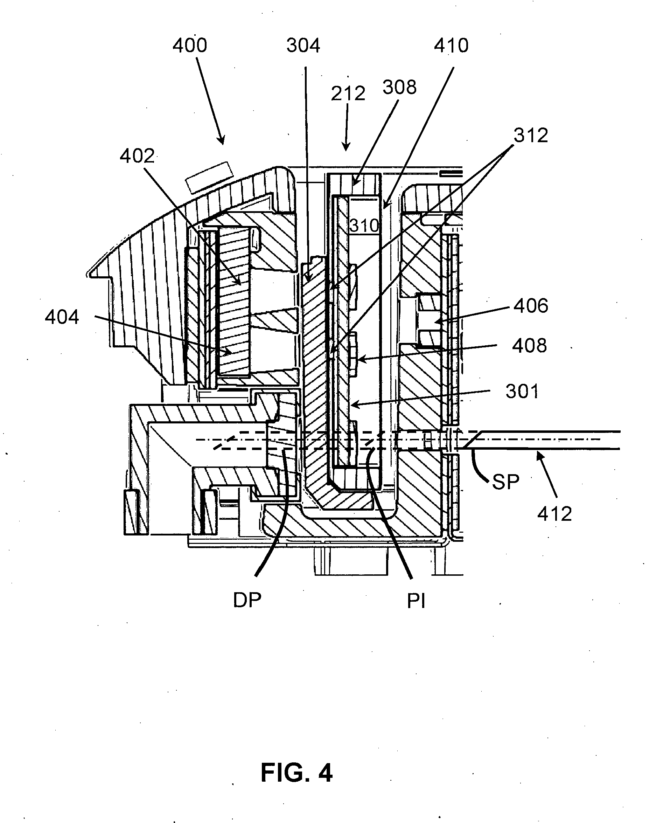

In an embodiment, the rotatable support 300 has a right circular cylinder shape of at least 80% of a hollow cylinder shape extending annularly around an axis which is, when the cartridge 202 is mounted in the station 200, the rotation axis A. The test support 301 may be a test strip. The rotatable support 300 may comprise an annular portion 302 and a cylindrical portion 304, which extends from an outermost radial extremity of the annular portion 302. The cylindrical portion 304, when in use, is lodged inside the annular chamber 212. The test supports 301 are positioned along the cylindrical portion 304, so that they can be selectively and/or successively scrolled in front of the injector and the analyzer. For instance, the test supports 301 are part of a holder 308, which comprises several chambers 310, separated from each other along a perimeter around the axis A. The plurality of chambers 310 are arranged next to one another in a right circular cylinder shape of at least 80% of a circle. To allow light to go through, the holder 308 includes at least one aperture 312 per chamber 310 (represented in the upper left zoom where the rotatable support is shown as transparent). The chambers 310 are all at an equal distance of the rotation axis A, so that the injector can selectively inject urine after the desired chamber is positioned at a desired location facing the injector. The injector may translate towards the chamber 310 and pierce a lid closing the chamber 310 (visible on Figure 4). A drain opening 314 is provided in the rotatable support 300 to allow evacuating urine from the injector to the outside of the device 100.

The annular portion 302 of the rotatable support 300 remains outside of the annular chamber 212 to strengthen the cylindrical portion and/or to drive in rotation the cartridge 202. To that end, the annular portion 302 may include a mechanical coupling 306, which cooperates with a shaft of the station 200.

The dimensions relative to the cartridge 202 are disclosed in WO'909, WO'933 and the above-mentioned French applications. The maximum dimension of the device 100 transversal to the rotational axis A is less than 15 cm, even less than 10 cm. The maximum dimension of the device along the rotation axis A is less than 5 cm.

Figure 4 shows in more detail the interaction between the cartridge 202 and the station 200 when or after the injector is activated. The analyzer 400 comprises at least one light source 402, 404 (e.g., two) and at least one optical sensor 406. Light travels from the light source 402, 404 to the optical sensor 406 while passing through the cartridge 202 and in particular the cylindrical portion 304, the aperture 312 of the holder 308, the test support 301 and thus the reagent 408. The injector includes an injection end 412 (for example a needle), which can be moved between several positions, which are represented in dash lines in Figure 4. In a standby position SP, the injection end 412 is outside the cartridge 202 (in an innermost position), so that the cartridge 202 can rotate freely in the annular housing 212; in an injection position IP, the injection end 412 has pierced the lid 410 to access the inside of the chamber 310 and may inject some urine on the test support 301; in the drain position DP, the injection end 412 is able to evacuate urine through the drain opening 314 of the rotatable support 300.

In position SP, the injector is located radially inward the annular chamber. This allows to maximize the radius of the annular chamber while minimizing the size of the station 200.

## OVERALL DESCRIPTION OF THE TEST STRIP

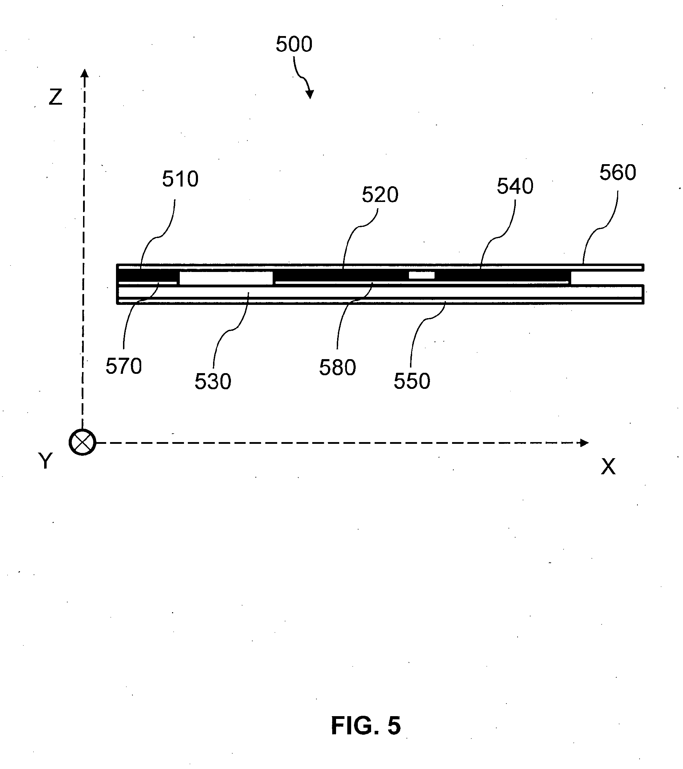

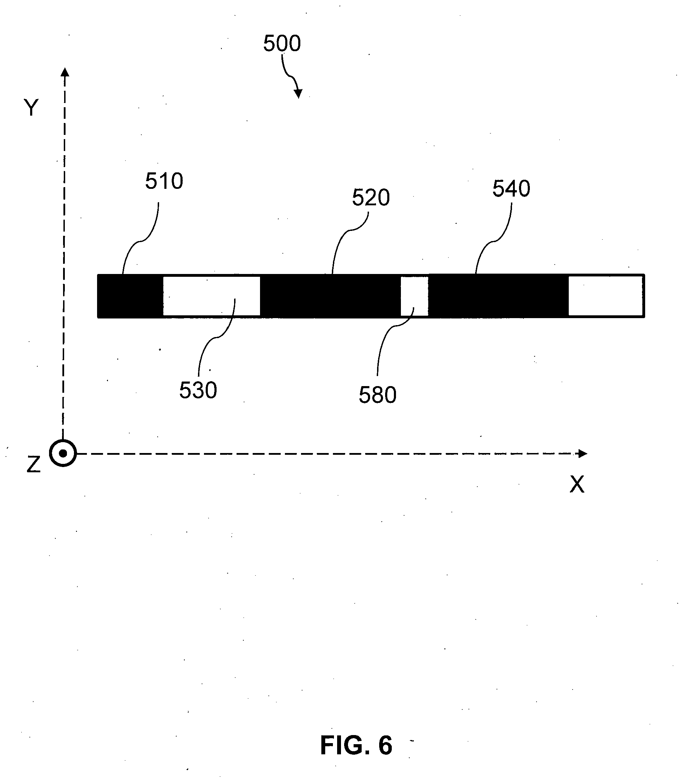

The test support 301 may be a test strip 500. As visible on Figure 5 and Figure 6, the test strip 500 extends mainly along a longitudinal direction X. In one embodiment, the test strip 500 has a generally parallelepipedic shape. The test strip 500 may present a width inferior to 5 mm, notably between 0.5 mm and 3 mm in a transversal direction Y, orthogonally to the longitudinal axis X. The test strip 500 may present a length inferior to 20 mm, notably between 10 mm and 15 mm, along the longitudinal axis X. The test strip 500 presents a thickness along an elevation axis Z that varies along the strip depending on its components. The test strip 500 comprises different components and materials that absorb and/or react to urine, and notably to the sodium in the urine or the pH of the urine.

To that end, the test strip 500 comprises a sample pad 510, a test pad 520 and a backing pad 530. The test strip 500 may further comprise a reference pad 540, an excitation optical filter 550 and/or an emission optical filter 560.

The sample pad 510 is arranged at an extremity of the test pad 500 along the longitudinal axis X. The sample pad 510 may extend on a length along the longitudinal axis X inferior to 4 mm.

The sample pad 510 is fixed to the backing pad 530 with at least an adhesive tape 570. The adhesive tape 570 is in particular a double-sided adhesive. The adhesive tape 570 is for example an acrylic based adhesive.

The sample pad 510 is configured to receive a urine sample. In particular, the sample pad 510 is configured to face the injector end 412 when the test strip 500 is arranged in a chamber 310 of the cartridge 202 and placed inside the analysis device 100. The sample pad 510 is configured to receive the urine injected by the injector on the test strip 500.

The sample pad 510 may be made of a porous plastic membrane, notably a PE (Polyéthylène) and PP (Polypropylène) hydrophilic membrane. By plastic it is understood, a synthetic or semisynthetic organic polymer, contrary to cellulose for example, which is an organic compound. As it will be explained in further detail above, such a material enables an efficient transport of the ions, and in particular sodium ions, across to the test strip 500.

In an embodiment, the sample pad 510 may comprise a buffer able to keep the pH at a value close to 7. In particular, the buffer is able to keep the pH between 6.5 and 7.5 for urine samples of pH 5-8. As it will be explained below, the dissociation constant (Kd) of the probe is notably dependent on the pH which is due to the pH dependance of the fluorescence intensity of the fluorophore. The urine of an individual is usually between 4.5 and 8, which would lead to important variations in the fluorescence response of the probe to sodium concentrations. Maintaining the pH near pH 7 ensures the measurements have a negligible pH dependence and enables a more accurate ion measurement. In the case of sodium detection, since the interest is to measure sodium ions in urine, the buffer should not contain sodium ions and if possible, potassium ions as well to reduce interference. Thus, buffers which are organic buffers without sodium salt are used. In particular, a Tris HCl buffer of 0.1 M to 1 M was used at a pH of 7.5. Sample pads were soaked in this buffer and dried in an oven between 35°C and 60°C.

The test pad 520 is arranged next to the sample pad 510, in particular between the sample pad 510 and the reference pad 540 along the longitudinal axis X. The test pad 520 may extend on a length along the longitudinal axis X inferior to 4 mm.

The test pad 520 is fixed to the backing pad 530 with at least an adhesive tape 580. The adhesive tape 580 is in particular a double-sided adhesive. The adhesive tape 580 is for example an acrylic based adhesive. The adhesive tape 580 fixing the test pad 20 may be the same as the adhesive tape 570 fixing the sample pas 510. In a variant, as illustrated in Figure 5, the adhesive tape 580 fixing the test pad 20 is different from the adhesive tape 570 fixing the sample pad 510.

The test pad 520 comprises the reagent 408. The ionophore may be from the channel former type. A channel forming ionophore is configured to form a channel by incorporating itself in a lipid membrane to transport ions. In particular, in case of sodium detection, the test pad 520 comprises a probe made of a fluorophore conjugated with a sodium ionophore. The probe may be in particular made of a crown ether linked to at least a fluorescein molecule.

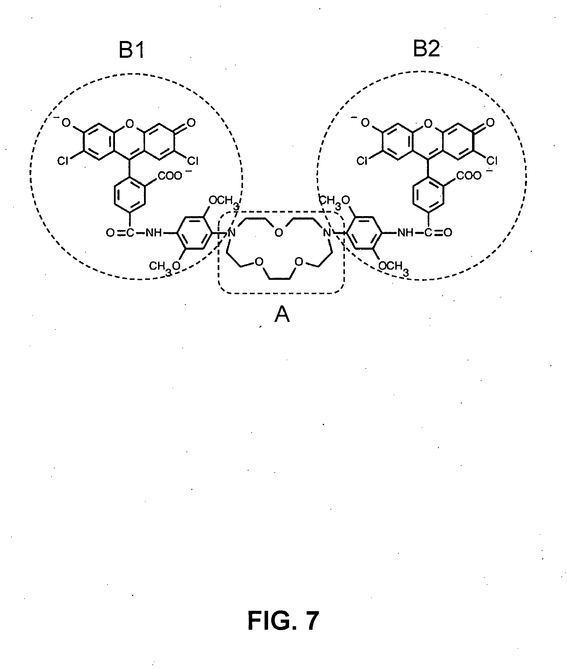

As illustrated on Figure 7, the probe is for example 5-[[4-[13-[4-[[3-carboxylato-4-(2,7-dichloro-3-oxido-6-oxoxanthen-9-yl)benzoyl]amino]-2,5-dimethoxyphenyl]-1,4,10-trioxa-7,13-diazacyclopentadec-7-yl]-2,5-dimethoxyphenyl]carbamoyl]-2-(2,7-dichloro-3-oxido-6-oxoxanthen-9-yl)benzoate, notably sold under the name "Sodium Green". In reference to Figure 7, the sodium ionophore is circled and referenced A and the fluorophore is circled and referenced B1 and B2. Sodium Green consists of a fluorescein analog linked to each of the nitrogen atoms of a crown ether with a cavity size that confers selectivity for the sodium ion.

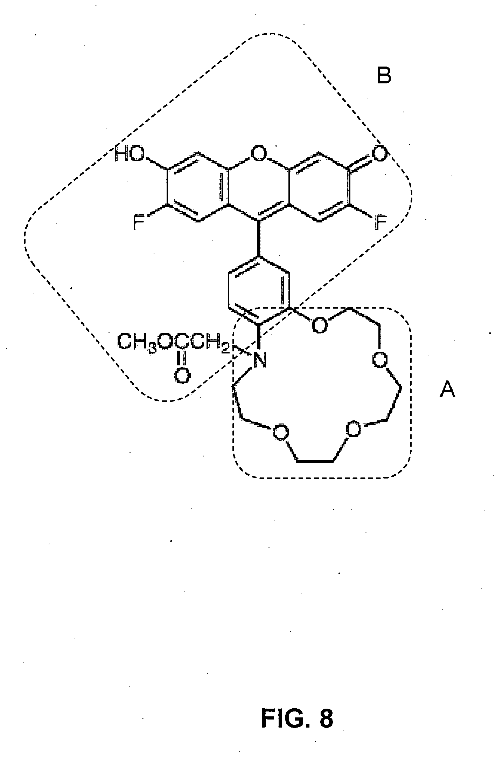

In a variant, in reference to Figure 8, the probe is for example made of a single fluorescein molecule linked to a crown ether. In reference to Figure 8, the sodium ionophore is circled and referenced A and the fluorophore is circled and referenced B. The ionophore is able to reversibly bind with, notably, sodium ions. Sodium ions are able to bind to the hydrophilic center of the ionophore and to form an ionophore-ion complex. The fluorophore is bonded to the ionophore and serves as a marker. In particular, the fluorescence emission intensity of the fluorophore increases when a sodium ion is binding with the ionophore.

In a variant, the ionophore may be from the ion carrier type. A carrier ionophore is a molecule with polar interior and a polar exterior that carry ions across by charge.

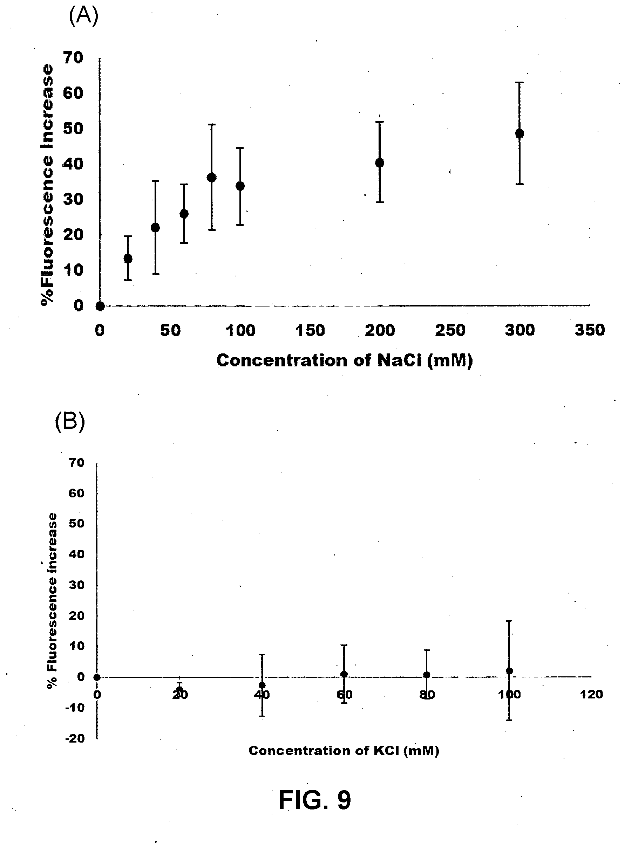

Figure 9 represents tests carried out by the inventors using a test strip 500 according to the invention with Sodium Green as a probe: with a NaCl solution (graph A) and with a KCl solution (graph B). Graph A shows a quasi-linear relationship in the range [0 mM - 60 mM] which is the range of expected concentrations for sodium in urine. In particular, a concentration below 20 mM is considered usually as a "low" sodium concentration, a concentration between 20 mM and 30 mM is considered a "normal" sodium concentration and a concentration above 40 mM is considered as a "high" sodium concentration. Graph B shows that potassium reacts relatively little with the probe. Therefore, Sodium Green presents a good specificity to sodium over potassium. Figure 9 illustrates therefore that the test strip 500 enables qualitative determination of sodium in urine as high or low but has significantly high errors and smaller range for accurate semi-quantitative or quantitative measurements.

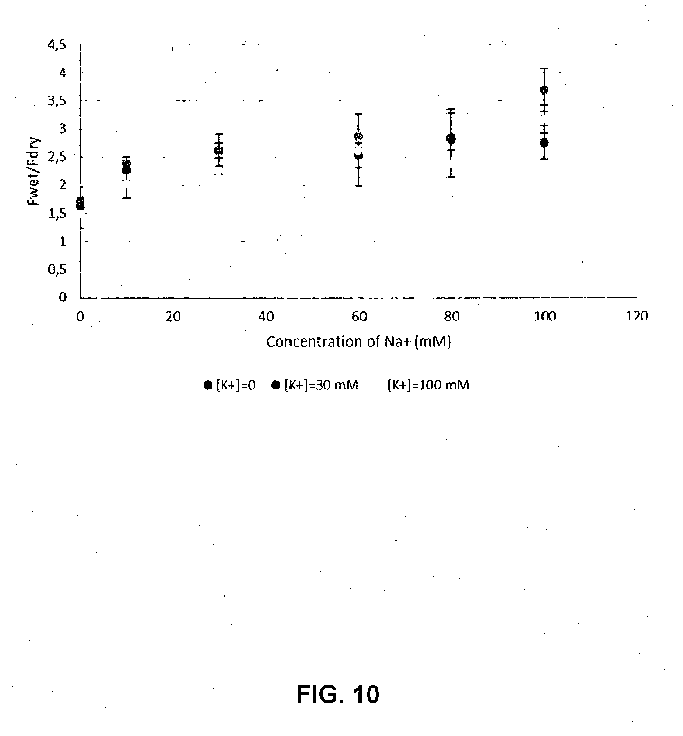

Figure 10 represents tests carried out by the inventors using a test strip 500 according to the invention with Sodium Green as a probe with a synthetic urine at different sodium and potassium concentrations. Figure 10 displays a "wet to dry ratio", i.e. the ratio of fluorescence of a strip with a sample comprising sodium with the fluorescence of a dry strip (without sample). Figure 10 confirms that the test strip 500 enables qualitative determination of sodium in urine.

Referring again to Figure 4, the test pad 520 is configured to face the light sources 402, 404 on one side and the optical sensor 406 on the other side when the test strip 500 is arranged in a chamber 310 of the cartridge 202 and placed inside the analysis device 100.

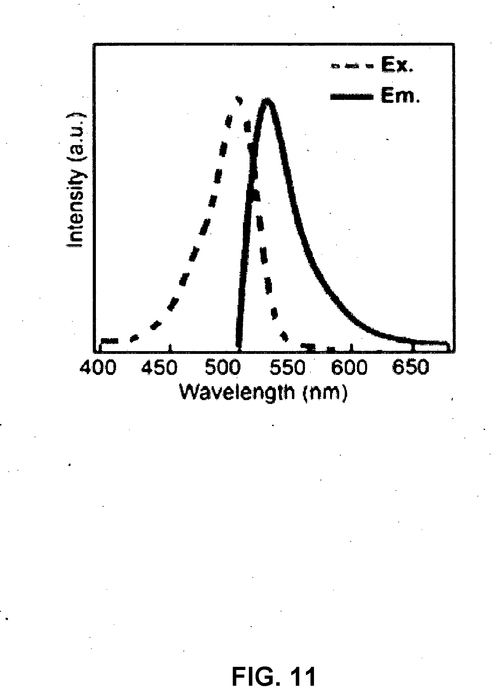

Figure 11 represents the intensity respectively regarding excitation and excitation of the probe as a function of the wavelength. For Sodium Green, the peak excitation and emission wavelengths are respectively 507 nm and 532 nm. For a probe present in an excess of sodium ions, free sodium ion concentration in solution can be calculated using the following equation:

$$[Na^+]_{free} = K_d \left[ \frac{F - F_{min}}{F_{max} - F} \right]$$

where F is the relative fluorescence reading, Fmin is the relative fluorescence reading in the absence of sodium and Fmax is the relative fluorescence of the sodium ion-saturated fluorophore. The equilibrium dissociation constant is a molecular property of the probe that is dependent on the solution conditions, but independent of the instrument used to quantify fluorescence. For the quantitation range of interest, the total sodium ion concentration is much greater than the fluorophore concentration, thus allowing for the assumption that the free sodium ion concentration is equal to the initial sodium ion concentration. In particular, the dissociation constant (Kd) for Sodium Green for Na+ is 6 mM at 22°C in potassium-free solution and above 21 mM at 22°C in solutions containing both Na+ and K+ (with a total ion concentration of 135 mM).

The probe is dried onto a paper substrate to form the test pad 520. The paper substrates were optimized based on optimal response to sodium after drying of the probe on the paper, low interference of reagents used in the paper preparation to the sodium response and homogeneity of the dried probe on the paper substrate. Cellulose based filter paper, cotton linter paper and high absorption cellulose namely guthrie paper were tested as potential candidates. Sodium Green probe was dispersed in DiMethyl SulfOxide (DMSO) to form a stock solution of 1mM to 5 mM. The probe was diluted to required concentration between 5 μM to 25 μM. The paper supports were soaked in this solution and dried between 35°C to 60°C. The dried paper was tested using National Institute of Standards and Technology (NIST) sodium standard solutions at 0.4 mM, 4 mM and 40 mM.

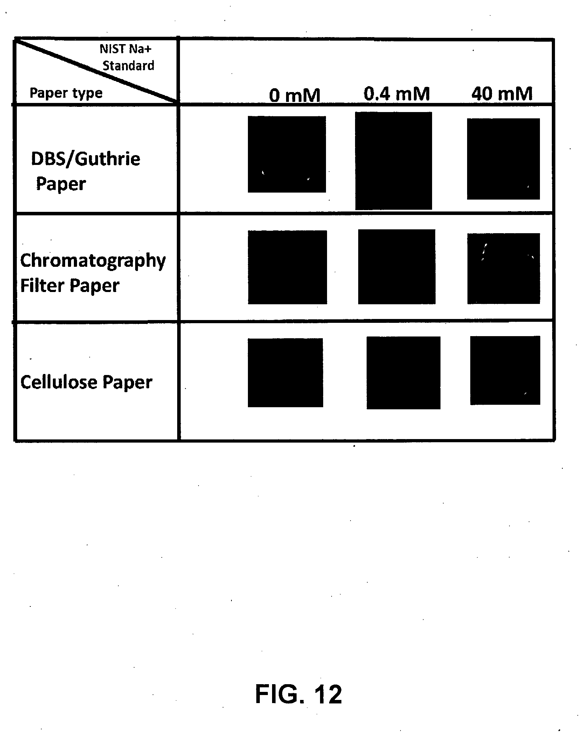

The results are illustrated on Figure 12. DBS/Guthrie paper performed better among the three tested paper types in terms of fluorescence response visualization and dispersion of the probe. In particular, the DBS/Guthrie paper shows a more consistent increase in intensity than the other two papers. In view of these tests, the test or reference pad is made of DBS/Guthrie paper, to obtain high absorption. Therefore, in a preferred embodiment, the test and reference pad substrate are advantageously made of a DBS/Guthrie paper.

The reference pad 540 is arranged on the backing pad 530. The reference pad 540 is arranged next to the test pad 520 along the longitudinal axis X. The reference pad 540 may extend on a length along the longitudinal axis X inferior to 4 mm.

The reference pad 540 is fixed to the backing pad 530 with at least an adhesive tape 580. The adhesive tape 580 is in particular a double-sided adhesive. The adhesive tape 580 is for example an acrylic based adhesive. The adhesive tape 580 fixing the reference pad 540 may be the same as the adhesive tape 580 fixing the test pad 520, as illustrated on Figure 5. In a variant, the adhesive tape 580 fixing the reference pad 540 is different from the adhesive tape 580 fixing the test pad 520.

The reference pad 540 may comprise the fluorophore without the ionophore. In particular, in case of a test pad containing Sodium Green, the reference pad 540 comprises fluorescein. The reference pad 540 material is similar to the test pad material.

Referring again to Figure 4, the reference pad 540 is configured to face the light sources 402, 404 on one side and the optical sensor 406 on the other side when the test strip 500 is arranged in a chamber 310 of the cartridge 202 and placed inside the analysis device 100. The reference pad 540 enables to correct the effects of components in urine which could influence the fluorescence of the fluorophore and in turn, the fluorescence emitted by the test pad 500.

The backing pad 530 is configured to support the sample pad 410, the test pad 420 and the reference pad 540. The backing pad 530 is configured to transport the sodium ions across to the test strip 500 from the sample pad 410 towards the test pad 410 and eventually to the reference pad 540.

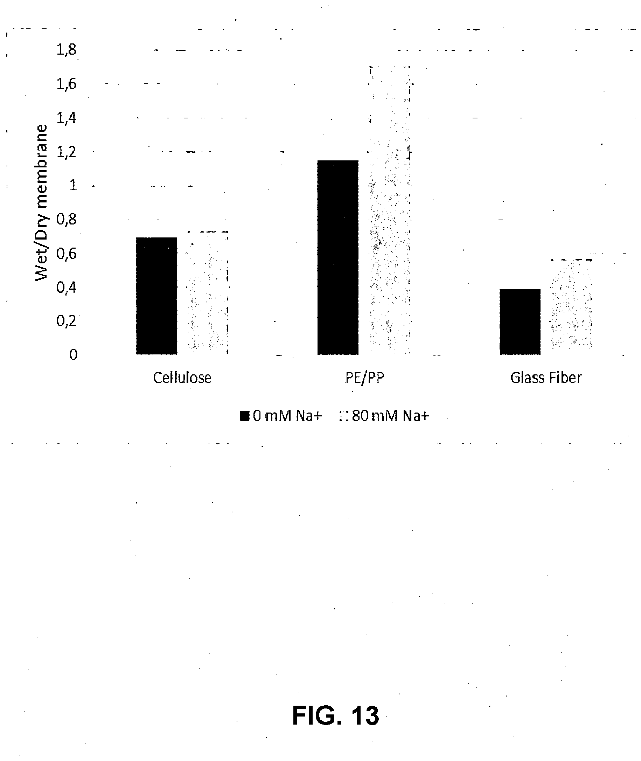

The backing pad 530 was optimized by the inventors to transport the ions across to the test pad 500. Conventional materials, for example cellulosic materials, cause the ions to bind to the membrane and therefore the sodium does not migrate or diffuse fast enough on these conventional materials. These materials are therefore not suited for use as the backing pad. The following tests have been carried out by the inventors: different materials have been tested for the backing pad 530 by comparing, on one hand, the ratio of fluorescence of a strip with a sample comprising 0 mM of sodium with the fluorescence of a dry strip (without sample) and, on the other hand, the ratio of fluorescence of a strip with a sample comprising 80 mM of sodium with the fluorescence of a dry strip. The three tested materials are a cellulose membrane, a PE and PP membrane and a glass fiber membrane. The results are illustrated on Figure 13. The PE and PP membrane graph shows a higher intensity for 80 mM as opposed to 0 mM allowing better differentiation and Na detection, compared to the other materials.

The backing pad 530 is made of a porous plastic membrane. In particular, in view of the previous results, the backing pad 530 is made of a PE and PP hydrophilic membrane. By porous, it is understood that the membrane presents a sufficient porosity to allow the flowing of the sample through the backing pad.

For the optical-analysis, the backing pad 530 is preferably transparent or at least does not hinder or prevent the optical analysis.

As visible on Figure 5, the excitation optical filter 550 is arranged preferably at the bottom of the test pad 520 along the elevation direction Z. As visible on Figure 5, the excitation optical filter 550 may extend on the whole length of the test strip 500 and may also cover the sampler pad 510. The excitation optical filter 550 is configured to selectively transmit the main excitation wavelength of the fluorophore. In the case of Sodium Green, excitation optical filter 550 is configured to selectively transmit wavelengths around 507 nm.

The emission optical filter 560 is arranged preferably on top of the backing pad 530, opposed to the excitation optical filter 550 along the elevation direction Z. The emission optical filter 560 is configured to selectively transmit the main emission wavelength of the fluorophore. In the case of Sodium Green, emission optical filter 560 is configured to selectively transmit wavelengths around 532 nm.

The addition of the optical filter(s) (only emission or both excitation and emission depending on the excitation source) on the test strip 500 rather than on the analysis device 100, and notably on the light sources 402, 404 or on the optical sensor 406, enables to have a single analysis device 100 capable of accepting cartridges with both colorimetric and fluorescent strips. In particular, it enables use with white LED or specific wavelength LED as light sources 402, 404.

The test strip 500 disclosed here may be used with the cartridge previously disclosed and the urine analysis device as it will be described below. They provide a cost efficient, manufacturing efficient, easily adaptable, and mass-production suitable solution to improve the quality of the optical analysis and in particular the sodium concentration measurements.

The test strip 500 has been described in detail here above in the case of sodium detection for clarity issues. The skilled person will understand that the test strip 500 according to the invention may be utilized in a similar way to detect potassium or magnesium.

In particular, in case of magnesium detection, the probe may be from the carrier type, for example a probe sold under the name "Magnesium green".

In case of potassium detection, the probe may be from the channel tupe, for example a probe sold under the name "FluxOR™ Potassium Ion Channel Assay".

## METHOD TO MANUFACTURING THE TEST STRIP

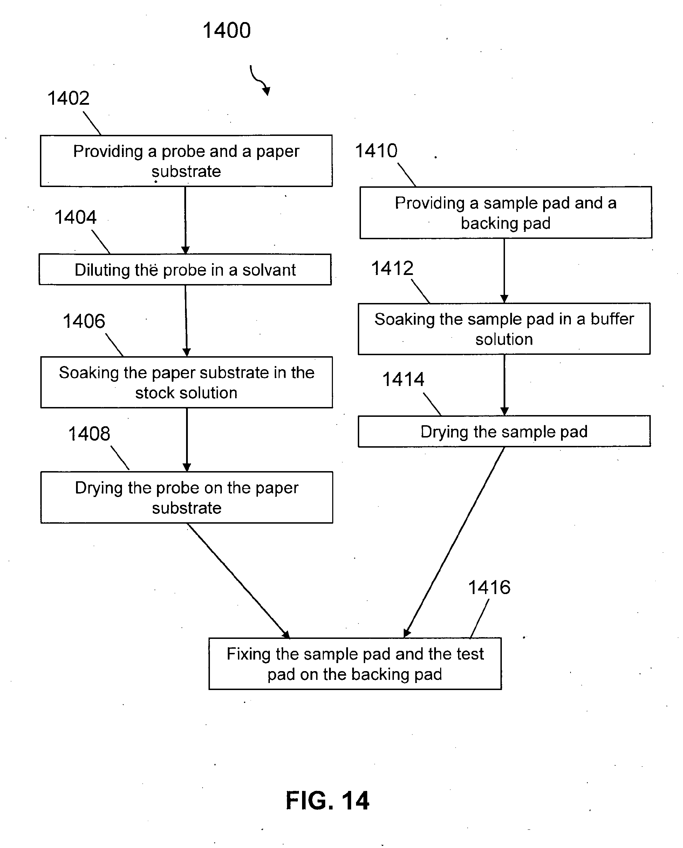

A method to manufacture a test strip 500 according to the invention will now be described in reference to Figure 14. The method is described for sodium detection but the skilled person in the art will understand that the method applies in a similar way to potassium or magnesium detection.

In step 1402, a probe and a paper substrate are provided. The probe is for example Sodium Green.

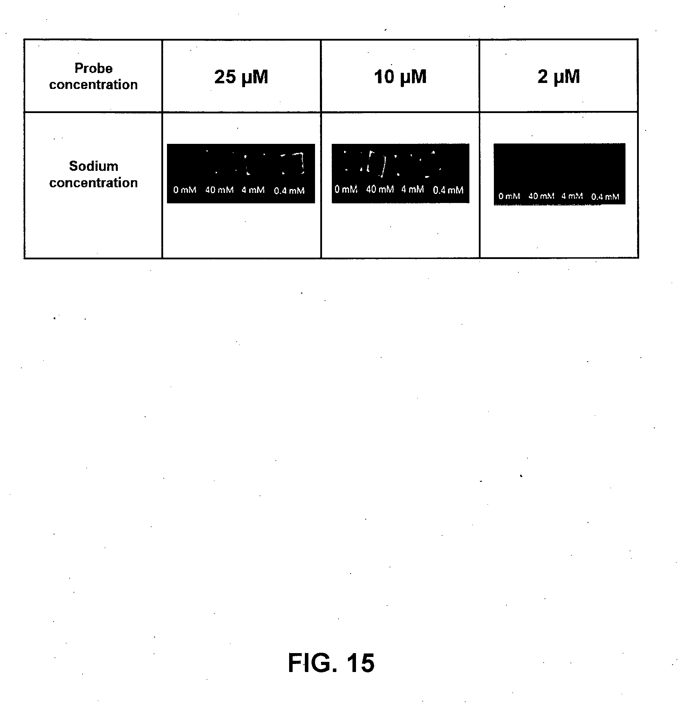

The inventors tested several probe concentrations. In particular, Sodium Green dispersed in DMSO was re-diluted in methanol to the required concentrations and test pads prepared and tested as described in 61. The fluorescence response was visualized for varying sodium and probe concentrations: the results are shown in Figure 15. The probe responds well at 10 $\mu$M with higher intensities at high concentration of sodium distinguishable from low concentration of sodium. At high probe concentration, the sample spot causes a ring effect which would require sample volume optimization in the test pad format. The probe concentration was optimized for test squares depending on the probe used for specific use cases. The substrate paper is notably DBS/Guthrie paper for sodium green probe while other substrates could be used to prepare strips depending on the use case. In strip format, the probe concentration of Sodium Green was between 25 $\mu$M and 50 $\mu$M for visualization on the optical system. This concentration is optimized on a case to case basis.

In step 1404, the probe is diluted in a solvent to form a stock solution. The inventors tested water and methanol as solvents to suspend the probe and methanol worked better than water for Sodium Green probe. Therefore, the solvent is advantageously methanol. For other ion specific probes, the solvent will be selected depending on the nature of the probe.

In step 1406, the paper substrate is soaked in the stock solution.

In step 1408, the probe is dried on the paper substrate by heating, in particular heated between 35°C and 60°C to form a test pad 510.

In step 1410, a sample pad 510 and a backing pad 530 are provided. Advantageously, a reference pad 540 is also provided.

In step 1412, the sample pad is soaked in a buffer solution containing 0.1M to 1 M Tris HCl at pH 7.5 for 10 to 20 mins.

In step 1414, the sample pad 510 and the test pad 520 are then dried in an oven between 35°C and 60°C.

In step 1416, the sample pad 510, the test pad 520 and eventually the reference pad 540 are fixed on the backing pad 530 with at least an adhesive layer 570, 580.

**OPERATION OF THE STATION AND THE TEST STRIP**

The operation of station 200 and the test strip 500 will now be described.

Station 200 is installed in toilets 102, as illustrated on Figure 1. A cartridge 202 is mounted in a removable manner in the station 200. The cartridge 202 comprises at least a test strip 500 as described above arranged in a chamber 310. Advantageously, the cartridge 202 further comprises a pH measuring strip arranged in at least a chamber 310.

An individual may urinate on station 200. A part of the urine stream is collected through the collection opening 218. Referring to Figure 4, the injector is initially in the standby position SP and the injection end 412 is outside the cartridge 202 (in an innermost position), so that the cartridge 202 can rotate freely in the annular housing 212. In particular, the test strip 500 is placed in front of the injector.

Then, the injector switches to the injection position IP and the injection end 412 pierces the lid 410 to access the inside of the chamber 310 and injects some urine on the sample pad 510.

The urine and the ions present in the urine flow through the test strip 500, in particular in the direction of the test pad 510 and the reference pad 540 thanks to the backing pad 530 made of a porous plastic membrane. The ions bind with the probe of the sample pad 510 and the intensity of fluorescence increases.

The station 200 then carries out an optical analysis. To that end, the light source 402, 404 illuminates test pad 510 and eventually the reference pad 540. Light travels from the light sources 402, 404 to the optical sensor 406 while passing through the cartridge 202 and in particular the cylindrical portion 304, the aperture 312 of the holder 308, the test strip 500 and in particular the test pad 520 and the reference pad 540.

The optical sensor 406 acquires optical data via the light emitted from the test strip 500. In particular, the optical sensor 406 analysis the variation of fluorescence of the test pad 510 and determine a concentration in the urine. The optical sensor 406 may use the variation of fluorescence of the reference pad 540 to correct the fluorescence emitted by the test pad 510.

When the sample pad 510 is not buffered to obtain a stabilized pH around pH 7, in a variant, the station 200 may carry out an additional pH measurement strip. To that end, the cartridge 202 is rotating and the pH measuring strip is placed in front of the light sources 402, 404. Another urine sample is injected on the pH measurement strip. In a known way, the color of the pH measurement strip is representative of the pH of the liquid injected on it. The optical sensor 406 analyses this color and determines the pH of the urine. The ion concentration is then corrected with the pH value of the urine as the reactivity of the probe depends on the pH as previously explained.

The station 200 may communicate remotely the measured ion concentration with a remote entity, such as smartphone 114 or a server 116. In a variant, the station 200 may communicate an indicator as "low", "normal" or "high" representative of the measured ion concentration compared to an expected sodium concentration for the individual. The individual may therefore easily and regularly monitor the ion of interest concentration in his or her urine.

**Claims**

1. A test strip (500) for detecting an ion of interest present in urine for a urine optical analysis device cartridge (202), the test strip (500) comprising:

   - a sample pad (510) configured to receive a urine.sample,
   - a test pad (520) comprising a probe made of a fluorophore conjugated with an ionophore, the fluorescence emission intensity of the fluorophore increasing when an ion of interest is binding with the ionophore, and
   - a backing pad (530) on which the sample pad (510) and the test pad (520) are arranged, the backing pad (530) being configured to transport the ions of interest across to the test strip (500) from the sample pad (510) towards the test pad (520), the backing pad (530) being made of a porous plastic membrane.

2. The test strip (500) according to claim 1, wherein the ion of interest is chosen between: sodium, potassium, and magnesium.

3. The test strip (500) according to claim 1 or 2, wherein the test strip (500) extends along a longitudinal direction (X), the test strip (500) presenting a length inferior to 20 mm, and/or a width inferior to 5 mm.

4. The test strip (500) according to any one of claims 1 to 3, wherein the test strip (500) further comprises an excitation optical filter (550), arranged preferably at the bottom of the test strip (500), the excitation optical filter (550) being configured to selectively transmit the main excitation wavelength of the fluorophore.

5. The test strip (500) according to any one of claims 1 to 4, wherein the test strip (500) further comprises an emission optical filter (560), arranged preferably on top of the test and reference pad, the emission optical filter (560) being configured to selectively transmit the main emission wavelength of the fluorophore.

6. The test strip (500) according to any one of claims 1 to 5, wherein the sample pad (510) is made of a porous plastic membrane.

7. The test strip (500) according to any one of claims 1 to 6, wherein the sample pad (510) comprises a buffer able to keep the pH close to 7, in particular between pH 6.5 and 7.5 for a urine sample of pH 5 to 8.

8. The test strip (500) according to any one of claims 1 to 7, wherein the test pad (520) is made of cellulose.

9. The test strip (500) according to any one of claims 1 to 8, wherein the backing pad (530) is made of a PE and PP hydrophilic membrane.

10. The test strip (500) according to any one of claims 1 to 9, wherein the ion of interest is sodium and the probe is made of a crown ether linked to a fluorophore.

11. The test strip (500) according to any one of claims 1 to 10, wherein the test strip (500) further comprises a reference pad (540) arranged on the backing pad (530), the reference pad (540) comprising the fluorophore without the ionophore.

12. A cartridge (202) for a urine optical analysis device (100), the cartridge (202) comprising a plurality of chambers (310) arranged next to one another in a right circular cylinder shape of at least 80% of a circle with a diameter comprised between 3 and 10 cm, wherein at least a chamber (310) comprises a test strip (500) according to any of claims 1 to 11.

13. The cartridge (202) according to claim 12, wherein the cartridge (202) further comprises a pH measuring strip arranged in at least a chamber (310).

14. A urine optical analysis device (100) comprising:

- the cartridge (202) according to claim 12 or 13,
- a station (200), configured to be positioned on a wall of a toilet bowl, the station comprising:

+ a case (204) comprising an annular housing (212) in which the cartridge is at least partially received and is rotatably mounted therein around a rotation axis (A),
+ an injector (412), configured to inject urine on the test strip (500),
+ an analyzer (400) with a light source (402, 404) and a light sensor (406), configured to emit and receive light, and to detect a change of fluorescence of the test strip (500).

15. A method (1400) to manufacture a test strip (500) according to any of claims 1 to 14, comprising:

- providing (1402) a probe and a paper substrate,
- providing (1410) a sample pad (510), and a backing pad (530) made of a porous plastic membrane,
- diluting (1404) the probe in a solvent to form a stock solution,
- soaking (1406) the paper substrate into the stock solution with shaking,
- drying (1408) the probe on the paper substrate by heating it to form a test pad,
- fix (1416) the sample pad and the test pad on the backing pad with an adhesive layer.

16. The method (1400) according to claim 15, wherein the solvent is methanol.

**FIG. 1**

100

A

204

206

200

202

216

212

210

218

208

204

**FIG. 2**

**FIG. 3**

**FIG. 4**

FIG. 5

**FIG. 6**

FIG. 7

**FIG. 8**

(A)

(B)

FIG. 9

**FIG. 10**

FIG. 11

| NIST Na+ Standard / Paper type | 0 mM | 0.4 mM | 40 mM |
|---|---|---|---|
| DBS/Guthrie Paper | ■ | ■ | ■ |
| Chromatography Filter Paper | ■ | ■ | ■ |
| Cellulose Paper | ■ | ■ | ■ |

## FIG. 12

**FIG. 13**

23

1400

1402

Providing a probe and a paper substrate

1404

Diluting the probe in a solvant

1406

Soaking the paper substrate in the stock solution

1408

Drying the probe on the paper substrate

1410

Providing a sample pad and a backing pad

1412

Soaking the sample pad in a buffer solution

1414

Drying the sample pad

1416

Fixing the sample pad and the test pad on the backing pad

FIG. 14

| Probe concentration | 25 µM | 10 µM | 2 µM |
|---|---|---|---|
| Sodium concentration | | | |

FIG. 15

Europäisches
Patentamt
European
Patent Office
Office européen
des brevets

# EUROPEAN SEARCH REPORT

Application Number

EP 22 31 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 115 379 804 A (VISENSE INC) 22 November 2022 (2022-11-22) * the whole document * | 1-16 | INV. A61B5/145 A61B5/20 A61B5/00 A61B10/00 G01N33/493 |
| Y | WO 2020/018232 A1 (BLOOM HEALTH INC [US]) 23 January 2020 (2020-01-23) * the whole document * * figure 7A * | 1-16 | |
| Y | WO 2020/097138 A1 (UNIV HOUSTON SYSTEM [US]) 14 May 2020 (2020-05-14) * the whole document * * figures 1-4B * * example 26 * | 1-16 | |
| Y | US 2019/343386 A1 (PULITZER JOVAN HUTTON [US] ET AL) 14 November 2019 (2019-11-14) * the whole document * | 1-16 | |
| Y | KLUCINSKA KATARZYNA ET AL: "Nanoparticles of Fluorescent Conjugated Polymers: Novel Ion-Selective Optodes", ANALYTICAL CHEMISTRY, vol. 88, no. 11, 11 May 2016 (2016-05-11), pages 5644-5648, XP093052327, US ISSN: 0003-2700, DOI: 10.1021/acs.analchem.6b00737 * the whole document * * abstract * * Sceme 1; figure 1 * | 1-16 | TECHNICAL FIELDS SEARCHED (IPC) A61B G01N |

–/–

The present search report has been drawn up for all claims

| Place of search | Date of completion of the search | Examiner |
|---|---|---|
| The Hague | 7 June 2023 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 31 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SAMBATH KARTHIK ET AL: "Potassium Ion Fluorescence Probes: Structures, Properties and Bioimaging", CHEMPHOTOCHEM, vol. 5, no. 4, 28 December 2020 (2020-12-28), pages 317-325, XP093052001, Hoboken, USA ISSN: 2367-0932, DOI: 10.1002/cptc.202000236 * the whole document * | 1-16 | |
| Y | PAK YEN LENG ET AL: "Conjugated polymer based fluorescent probes for metal ions", COORDINATION CHEMISTRY REVIEWS, vol. 433, 1 April 2021 (2021-04-01), page 213745, XP093051932, NL ISSN: 0010-8545, DOI: 10.1016/j.ccr.2020.213745 * the whole document * | 1-16 | |
| Y | NOVIANA EKA ET AL: "Microfluidic Paper-Based Analytical Devices: From Design to Applications", CHEMICAL REVIEWS, vol. 121, no. 19, 13 October 2021 (2021-10-13), pages 11835-11885, XP055854824, US ISSN: 0009-2665, DOI: 10.1021/acs.chemrev.0c01335 * the whole document * | 1-16 | **TECHNICAL FIELDS SEARCHED (IPC)** |
| Y | EP 0 861 843 A2 (MINNESOTA MINING & MFG [US]) 2 September 1998 (1998-09-02) * the whole document * | 1-16 | |

-/--

The present search report has been drawn up for all claims

| Place of search | Date of completion of the search | Examiner |
|---|---|---|
| The Hague | 7 June 2023 | Boiangiu, Clara |

Europäisches Patentamt

European Patent Office

Office européen des brevets

# EUROPEAN SEARCH REPORT

Application Number

EP 22 31 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2021/175909 A2 (WITHINGS [FR]) 10 September 2021 (2021-09-10) * the whole document * | 1-16 | |
| Y | Nerea De Acha ET AL: "Fluorescent Sensors for the Detection of Heavy Metal Ions in Aqueous Media", Sensors, 31 January 2019 (2019-01-31), pages 1-32, XP093051777, Retrieved from the Internet: URL:https://www.ncbi.nlm.nih.gov/pmc/articles/PMC6386841/pdf/sensors-19-00599.pdf [retrieved on 2023-06-05] * the whole document * | 1-16 | |
| Y | US 2018/275088 A1 (HUFF JEFFREY B [US] ET AL) 27 September 2018 (2018-09-27) * the whole document * | 1-16 | |
| Y | Karl Cammann ET AL: "Chemical and Biochemical Sensors" In: "Ullmann's Encyclopedia of Industrial Chemistry", 15 January 2001 (2001-01-15), Wiley-VCH, Weinheim, XP055417528, ISBN: 978-3-527-30673-2 DOI: 10.1002/14356007.b06_121, * the whole document * | 1-16 | TECHNICAL FIELDS SEARCHED (IPC) |
| Y | US 2019/062813 A1 (AMIN HIMANSHU S [US]) 28 February 2019 (2019-02-28) * the whole document * | 1-16 | |
| Y | FR 3 115 365 A1 (WITHINGS [FR]) 22 April 2022 (2022-04-22) * the whole document * | 1-16 | |

The present search report has been drawn up for all claims

| Place of search | Date of completion of the search | Examiner |
|---|---|---|
| The Hague | 7 June 2023 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 31 5335

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2023

| Patent document cited in search report | | Publication date | Patent family member(s) | | Publication date |
|---|---|---|---|---|---|
| CN 115379804 | A | 22-11-2022 | CN 115379804 A | | 22-11-2022 |
| | | | EP 4087496 A2 | | 16-11-2022 |
| | | | US 2023105892 A1 | | 06-04-2023 |
| WO 2020018232 | A1 | 23-01-2020 | EP 3823524 A1 | | 26-05-2021 |
| | | | US 10383606 B1 | | 20-08-2019 |
| | | | US 2020015791 A1 | | 16-01-2020 |
| | | | WO 2020018232 A1 | | 23-01-2020 |
| WO 2020097138 | A1 | 14-05-2020 | CA 3118540 A1 | | 14-05-2020 |
| | | | EP 3877753 A1 | | 15-09-2021 |
| | | | JP 2022511679 A | | 01-02-2022 |
| | | | KR 20210106417 A | | 30-08-2021 |
| | | | US 2022011315 A1 | | 13-01-2022 |
| | | | WO 2020097138 A1 | | 14-05-2020 |
| US 2019343386 | A1 | 14-11-2019 | US 2019343386 A1 | | 14-11-2019 |
| | | | US 2021068660 A1 | | 11-03-2021 |
| | | | WO 2019222081 A1 | | 21-11-2019 |
| EP 0861843 | A2 | 02-09-1998 | DE 69824985 T2 | | 25-08-2005 |
| | | | EP 0861843 A2 | | 02-09-1998 |
| | | | JP 4472795 B2 | | 02-06-2010 |
| | | | JP H10316683 A | | 02-12-1998 |
| | | | US 5958782 A | | 28-09-1999 |
| WO 2021175909 | A2 | 10-09-2021 | FR 3107823 A1 | | 10-09-2021 |
| | | | WO 2021175909 A2 | | 10-09-2021 |
| US 2018275088 | A1 | 27-09-2018 | BR 112019006930 A2 | | 02-07-2019 |
| | | | CN 109863391 A | | 07-06-2019 |
| | | | EP 3523640 A1 | | 14-08-2019 |
| | | | JP 2020502478 A | | 23-01-2020 |
| | | | JP 2022058772 A | | 12-04-2022 |
| | | | US 2018275088 A1 | | 27-09-2018 |
| | | | US 2021325334 A1 | | 21-10-2021 |
| | | | WO 2018067878 A1 | | 12-04-2018 |
| | | | ZA 201901985 B | | 18-12-2019 |
| US 2019062813 | A1 | 28-02-2019 | NONE | | |
| FR 3115365 | A1 | 22-04-2022 | FR 3115365 A1 | | 22-04-2022 |
| | | | WO 2022084622 A1 | | 28-04-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021175909 A
- WO 2021175944 A
- WO 909 A
- WO 944 A
- FR 2109383

- FR 2109384
- FR 2109391
- FR 2109392
- WO 933 A

### Non-patent literature cited in the description

- *Global sodium consumption and death from cardiovascular causes, https://pubmed.ncbi.nlm.nih.gov/25119608/*
- *Use of Urine Biomarkers to Assess Sodium Intake: Challenges and Opportunities, https://www.ncbi.nlm.nih.gov/pmc/articles/PMC5497310*

- **GHADERINEZHAD, F. ; CEYLAN KOYDEMIR, H. ; TSENG, D. et al.** Sensing of electrolytes in urine using a miniaturized paper-based device. *Sci Rep,* 2020, vol. 10, 13620, https://doi.org/10.1038/s41598-020-70456-6